# EUROPEAN PATENT SPECIFICATION

(11) **EP 2 623 109 B1**
(45) Date of publication and mention of the grant of the patent: **25.10.2017**
(21) Application number: 11829241.6
(22) Date of filing: 29.09.2011
(51) Int. Cl.: A23L 33/00, A23L 33/185, A23L 33/18

(54) **ENTERAL NUTRIENT**
ENTERALER NÄHRSTOFF
NUTRIANT ENTÉRAL

(30) Priority: 30.09.2010 JP 2010222796
(43) Date of publication of application: 07.08.2013
(73) Proprietor: Terumo Kabushiki Kaisha, Tokyo 151-0072 (JP)
(72) Inventor: TANI Yasuyo, Nakakoma-gun Yamanashi 409-3853 (JP); ARIIZUMI Tsuyoshi, Nakakoma-gun Yamanashi 409-3853 (JP)
(74) Representative: TBK
(86) International application number: PCT/JP2011/072309
(87) International publication number: WO 2012/043688

(56) References cited:
- EP-A1- 1 512 328
- WO-A1-2007/066694
- JP-A- 2007 126 379
- JP-A- 2009 274 964
- JP-A- 2010 083 774
- US-A- 5 330 972
- US-A1- 2009 280 217
- FUMINORI GODA ET AL.: '[Jomyaku·Keicho Eiyo UPDATA2009] PEG Hankokeika Eiyozai no Genjo to Kongo no Kadai' RINSHO EIYO vol. 114, no. 6, May 2009, pages 683 - 688

## Description

### Technical Field

This invention relates to an enteral nutrient formulated with protein, fat, carbohydrate, vitamin and mineral and having pH of 3.0 to 4.5.

### Background Art

Generally, protein has tendencies of coagulation, aggregation (i.e. a state of separation into supernatant and precipitation) and precipitation under acidic conditions. Accordingly, in the preparation of an acidic protein containing drink, it becomes necessary to solve these problems. In order to cope with these problems, there has been hitherto proposed a method for stabilization by using an acid-resistant milk serum protein as a protein source and by using a stabilizing agent such as a pectin, a water-soluble soybean polysaccharide or the like (Patent Document 1). Moreover, there has been proposed a stable preparation technique with respect to the case where a soy protein-containing solution is soybean milk or a peptide excellent in solubility (Patent Document 2). However, these there has never been reported technologies of stabilizing a protein solution containing protein derived from milk or soybean milk, which stabilizes protein derived from soy per se when formulated in a conventional nutrient.

Milk serum proteins that have been frequently employed in acidic enteral nutrients and are excellent in acid resistance are resistant to acid, but with the drawback that they are poorer in heat resistance than other types of protein materials. This causes inhomogeneity as a result of an abrupt increase in viscosity ascribed to the aggregation or thermal denaturation occurring by heating for sterilization or formulation step thereby influencing the physical properties of the resulting product. Although no health problem is involved, tube clogging may occur in some cases when such a nutrient is administered via a tube.

On the other hand, since it has been recently considered well-balanced to take both animal proteins and plant proteins, soy protein-containing drinks have established a great market owing to increasing number of health-oriented consumers.

However, plant proteins including soy proteins are more liable to suffer an influence, such as of minerals and the like contained in nutrients in large amounts, than milk-derived proteins, with concern that they aggregate under long-term storage thereby providing a rough taste and texture on the throat. Since there are some consumers who do not like a bitter taste inherent to soybean, there has been demanded an improvement in texture and taste along with the enhancement of physical properties and stability.

Patent Document 1: JP-A-2005-245217
Patent Document 2: JP-A-2006-61139

### Disclosure of Invention

### Problems to be Solved by the Invention

The present invention is to provide an enteral nutrient in which although a soy protein is contained as a protein source, no problem is involved in quality with respect to aggregation and viscosity abnormality and which shows a good taste without giving any sense of bitterness and coarseness on the throat inherent to soybean is good at taste without evoking a bitter taste inherent to soybean and a rough texture. Moreover, the present invention is also to provide an enteral nutrient wherein if a milk serum protein is contained, it is so stable as not to cause increase in viscosity and inhomogeneity due to aggregation or thermal denaturation by heating for sterilization or formulation step.

### Means for Solving the Problems

The above problems can be achieved by the following inventions.

(1) The present invention is directed to an enteral nutrient which comprises a protein, a fat, a carbohydrate, a vitamin and a mineral and has a pH of 3.0 to 4.5, characterized in that said protein includes 5 to 65%, relative to the total protein, of a soy protein containing 20 to 40% of a peptide fraction having a molecular weight of less than 6000. As used herein, the content (%) of peptide fraction having a molecular weight of less than 6000 in the soy protein is indicated by peak area percentage (column used: TSK gel G2500PW_{XL}, (Tosoh Corporation)) based on a high performance liquid chromatograph for determining a molecular weight distribution of soy protein as will be described hereinafter.
(2) The present invention is directed to an enteral nutrient set forth in the (1) above, which has an average particle size of not larger than 10 µm and is capable of being permeated through a 50 µm filter.
(3) The present invention is directed to an enteral nutrient set forth in the (1) or (2), which has a viscosity after thermal sterilization of 1000 to 30000 mPa·s.

### Effects of the Invention

According to the present invention, there can be provided an enteral nutrient that is inexpensive, rich in nutrients, is free of a problem on qualities such as of aggregation, viscosity abnormality and the like, and is good at taste and acidic in nature. Moreover, because of reduced degrees of aggregation and rough feeling and of a good taste, there can be provided an enteral nutrient which is acceptable from the standpoints of food preferences related to smooth texture on the throat, good texture and a long-lasting feeling of satiety.

### Mode for Carrying Out the Invention

The enteral nutrient of the present invention will be described in detail. The enteral nutrient of the present invention is obtained by formulating, in total proteins, a soy protein having a peptide fraction having a molecular weight of less than 6000 in an amount of 20 to 40%, preferably 25 to 40%, more preferably 25 to 35% and much more preferably 27 to 32%. If the content of the peptide fraction having a molecular weight of less than 6000 in the soy protein is less than 20%, aggregates would result after sterilization by heating in the course of preparation, leading to a poor taste owing to feeling of roughness. On the other hand, if the content of the peptide fraction having a molecular weight of less than 6000 in the soy protein is larger than 40%, bitterness would become intense, worsening the taste.

The amount of the soy protein having 20 to 40% of a peptide fraction having a molecular weight of less than 6000 to be formulated is 5 to 65 wt%, preferably 10 to 60 wt%, more preferably 20 to 50 wt% and much more preferably 20 to 40 wt%, relative to the total protein. A smaller amount would be nutritionally insufficient in terms of protein source. A soy protein contains a large amount of arginine, which forms an important component of nitrogen monoxide in vivo, prevents arterial sclerosis and has a role of eliminating active oxygen. When taking an amino acid balance into consideration, its amount in the total proteins is not less than 5 wt%, preferably not less than 10% and more preferably not less than 20%. If the formulated amount in the total proteins is larger than 60 wt%, bitterness would become intense thereby worsening the taste.

For the soy protein containing 20 to 40% of a peptide fraction having a molecular weight of less than 6000, there can be used, as a soy-derived material, soy milk, concentrated soy protein, or soy protein isolate, defatted soybean, soy whey protein and the like. Among these, soy protein isolate can be preferably used. These soybean-derived materials can be treated by an enzyme treatment method conventionally employed for food processing, e.g., treated by use of carboxyprotease (animal-derived pepsin or the like) at a temperature of 30°C to 60°C at pH of 5 to 10 generally for about 5 minutes to 24 hours although depending on the activity and amount of an employed proteinase to obtain a hydrolyzed soy protein solution. Thereafter, the solution is subjected to centrifugal separation to separate and remove insoluble matters and thermally sterilized, followed by hermetically sealed storage as it is or after concentration, or drying and powdering for practical usage. It will be noted that as a commercial product of soy protein containing about 30% of a peptide fraction having a molecular weight of less than 6000, there can be used Proleena (registered trade name) 900 (Fuji Oil Co., Ltd.).

In the present invention, the molecular weight distribution of soy protein is determined by dissolving 0.01 g of soy protein in a solvent (water : acetonitrile : trifluoroacetic acid = 55:45:0.1) and standing the resultant solution at room temperature overnight, and filtrating the solution with a membrane filter having a pore size of 0.45 µm to obtain a solution, and subjecting the solution to measurement under the following high performance liquid chromatographic measuring conditions of high-performance liquid chromatographic apparatus: Shodex GPC-101 (Showa Denko K.K.), detector: Ultraviolet Spectroaltimeter UV-41 (Showa Denko K.K.), column: TSK gel G2500PW_{XL}, ϕ7.8 mm × 300 mm (Tosoh Corporation), mobile phase: solvent (water : acetonitrile : trifluoroacetic acid = 55:45:0.1, column temperature: 40°C, flow rate: 0.5 ml/minute, measuring wavelength: 220 nm and injection amount: 20 µl.

In the present invention, the soy protein preferably shows pH in the range of 6.0 to 8.0 when dissolved in water.

In the enteral nutrient of the present invention, a protein and a peptid as the protein may be formulated, aside from the soy protein. Those other than the soy protein may include milk proteins such as casein, thick milk protein (TMP) and the like, animal proteins such as of egg albumen, collagen, protamine and the like, peptides prepared from these animal proteins, plant proteins such as of wheat, corn and the like, and peptides prepared from these plant proteins, of which milk proteins are preferred.

The enteral nutrient of the present invention may be formulated with an amino acid. As the amino acid, various amino acids including essential amino acids and non-essential amino acids may be cited. More particularly, of isoleucine, leucine, valine, lysine, methionine, phenylalanine, threonine, triptophan, arginine, histidine, glycine, alanine, proline, aspartic acid, serine, tyrosine, glutamic acid, cysteine, taurine, carnitine, ornithine and the like may be cited, for example. These amino acids may not always be contained as a free amino acid, but may be formulated in the form of inorganic acid salts (e.g. L-lysine hydrochloride, etc.), organic acid salts (e.g. L-lysine acetate, L-lysine malate, etc.), in vivo hydrolyzable esters (e.g. L-tyrosine methyl ester, L-methionine methyl ester, L-methionine ethyl ester, etc.), and N-substituted products (e.g. N-acetyl-L-tryptophan, N-acetyl-L-cysteine, N-acetyl-L-proline, etc.).

For the carbohydrate used in the enteral nutrient of the present invention, various carbohydrates may be formulated. For example, starch, dextrin, maltodextrin and the like may be cited, for example. Among these, dextrin with a low degree of hydrolysis is preferred and its use enables osmotic diarrhea to be prevented. One having DE (Dextrose Equivalent) ranging 8 to 25 is preferred, within which fluidity can be secured and osmotic pressure can be made adequately low. Not less than one or several types of dextrins chosen from among those mentioned above may be used in combinations. The DE of dextrin means a degree of hydrolysis of dextrin, and is expressed by the equation of DE = [(direct reducing sugar) (calculated as glucose)]/(solid content) × 100. For determining DE of dextrin, a conventional technique and its knowledge in this technical field or an appropriately modified form thereof can be applied, for which the Somogyi method can be typically mentioned.

The dextrins used in the enteral nutrients of the present invention may be one prepared according to conventional methods. More particularly, there may be used any of dextrin obtained by acid decomposition of starch and dextrin obtained by treatment with an enzyme such as α-amylase. Starch used as a starting material for dextrin may be derived from any origins, for which starches such as of corn, potato, sweet potato, waxy corn, waxy rice, waxy milo, tapioca and the like can be used, and not less than one to several types of starting materials selected therefrom may be used in combination.

The acidic enteral nutrient of the present invention may be further formulated with carbohydrates other than dextrin and including, for example, monosaccharides, disaccharides and oligosaccharides. More specifically, as a monosaccharide, glucose, fructose, galactose, sugar alcohol, mannitol, xylitol, inositol, sorbitol and the like may be cited. Also as a disaccharide, sucrose, lactose, maltose, trehalose and the like may be cited. Oligosaccharides may include polymers of about 3 to 6 units of the above monosaccharides.

Cluster dextrin and cyclodextrin may also be added. Besides, as a carbohydrate, there may be partly formulated a monosaccharide such as glucose, fructose or galactose, xylitol, sugar alcohol, arabinose, inositol, sorbitol or the like and a disaccharide such as sucrose, lactose, maltose, trehalose, palatinose or the like. For the purpose of improving an enteral environment, there may be added fructooligosaccharides, milk oligosaccharides, isomaltooligosaccharides, lactuloses and the like.

As a fat used in the enteral nutrient of the present invention, there can be used fats that have been usually in edible use. Examples include plant oils and fats such as linseed oil, perilla oil (egoma oil), olive oil, sesame oil, rice bran oil, safflower oil, perilla oil (shiso oil), soybean oil, corn oil, rape seed oil, germ oil, palm oil, palm kernel oil, castor oil, cotton seed oil, coconut oil, peanut oil and the like, animal oils and fats such as fish oil, milk fat and the like, medium-chain fatty acids, highly unsaturated fatty acids and the like, which may be used singly or in combination. Besides, processing additives such as DHA, EPA, diacylglycerol and the like may be added.

As a food fiber used in the enteral nutrient of the present invention, cellulose, lignin, indigestible dextrin, resistant starch, polydextrose, gum arabic, guar gum hydrolyzate, wheat bran, soy fiber, chitosan, chitin, Psyllium and the like may be cited, and one or two or more ingredients thereof may be formulated.

With respect to the electrolytes, minerals and trace elements used in the enteral nutrient of the present invention, sodium, potassium, calcium, magnesium and phosphorus may be cited for the electrolyte and mineral, and iron, copper, zinc, manganese, selenium, iodine, chromium and molybdenum may be cited for the trace element. It is preferred to formulate a plurality thereof in combination if possible. These may be formulated in the form of an inorganic electrolyte component or may be formulated as an organic electrolyte component. As the inorganic electrolyte component, alkali metal or alkaline earth metal salts such as chloride, sulfate, carbonate, phosphate and the like may be cited. As the organic electrolyte component, organic acids including, for example, citric acid, lactic acid, amino acids (e.g. glutamic acid, aspartic acid and the like), alginic acid, malic acid or gluconic acid may be cited. For the trace element, there may be used trace element-containing microorganism strain, which are derived from microorganisms (such as yeast) capable of accumulating a trace element and obtained by cultivation in a medium containing a highly concentrated trace element compound.

The amounts of the electrolyte, mineral and trace element are conveniently in the range, per 100 ml of the enteral nutrient, of 5 to 6000 mg, preferably 10 to 3500 mg for sodium, 1 to 3500 mg, preferably 25 to 1800 mg for potassium, 1 to 740 mg, preferably 25 to 300 mg for magnesium, 10 to 2300 mg, preferably 250 to 600 mg for calcium, 1 to 3500 mg, preferably 25 to 1500 mg for phosphorus, 0.1 to 55 mg, preferably 1 to 10 mg for iron, 0.01 to 10 mg, preferably 0.1 to 6 mg for copper, 0.1 to 30 mg, preferably 1 to 15 mg for zinc, 0.1 to 11 mg, preferably 0.1 to 4 mg for manganese, 0.1 to 450 µg, preferably 1 to 35 µg for selenium, 0.1 to 40 µg, preferably 1 to 35 µg for chromium, 0.1 to 3000 µg, preferably 1 to 150 µg for iodine, and 0.1 to 320 µg, preferably 1 to 25 µg for molybdenum.

Vitamins used in the enteral nutrient of the present invention include vitamin B1, vitamin B2, vitamin B6, vitamin B12, niacin, pantothenic acid, biotin, folic acid, vitamin C, vitamin A, vitamin D, vitamin E, vitamin K and the like, and it is preferred to formulate a possible plurality thereof in combination if possible. A vitamin derivative may be used as the vitamin.

The amount of the vitamin is conveniently in the range, per 100 ml of the enteral nutrient, of 0.1 to 40 mg, preferably 0.3 to 25 mg for vitamin B1, 0.1 to 20 mg, preferably 0.33 to 12 mg for vitamin B2, 0.1 to 60 mg, preferably 0.3 to 10 mg for vitamin B6, 0.1 to 100 µg, preferably 0.60 to 60 µg for vitamin B12, 1 to 300 mg, preferably 3.3 to 60 mg for niacin, 0.1 to 55 mg, preferably 1.65 to 30 mg for pantothenic acid, 1 to 1000 µg, preferably 14 to 500 µg for biotin, 10 to 1000 µg, preferably 60 to 200 µg for folic acid, 10 to 2000 mg, preferably 24 to 1000 mg for vitamin C, 0 to 3000 µg, preferably 135 to 600 µg for vitamin A, 0.1 to 50 µg, preferably 1.5 to 5.0 µg for vitamin D, 1 to 800 mg, preferably 2.4 to 150 mg for vitamin E, and 0.5 to 1000 µg, preferably 2 to 700 µg for vitamin K, respectively.

The pH of the enteral nutrient of the present invention ranges 3 to 4, preferably 3.4 to 3.8. If the pH is lower than 3, acidity would become so strong as to worsen the taste. On the other hand, when the pH is higher than 4 which promotes propagation of microorganism, bacteria would be incorporated during preparation, under which in case where thermal sterilization is insufficient, hygienic problems would be caused.

The enteral nutrient of the present invention has a viscosity of 1000 to 30000 mPa·s, preferably 2000 to 24000 mPa·s and much more preferably 4000 to 22500 mPa·s. If the viscosity is higher than 30000 mPa·s, tube fluidity would become worsened at the time of administration of the enteral nutrient, making the administration difficult. On the other hand, a viscosity equal to or higher than 1000 mPa·s that is close to a viscosity of water can ensure excellent tube fluidity, thus resulting in the unlikelihood of tube clogging. It will be noted that viscosity can be measured according to "Second Method Rotary Viscometer Method" of "28. Viscosity Measuring Method" in "B. General Test Procedures" of "the Japanese Standards of Food Additives Seventh Edition." For example, the measurement can be made by use of RB80L viscometer (Toki Sangyo Co., Ltd.).

The caloric value per gram of the enteral nutrient of the present invention is 0.6 to 2.0 kcal, preferably 0.7 to 1.5 kcal. If the caloric value per gram is less than 0.6 kcal, a satisfactory caloric value could not be nutritionally obtained. On the other hand, when the caloric value per gram is higher than 2.0 kcal, the osmotic pressure of the enteral nutrient after thermal sterilization would become too high, to increase frequency of diarrhea.

The enteral nutrient of the present invention has been illustrated hereinabove and should not be construed as limited thereto. If necessary, other ingredients and additives may be further added. For instance, additives employed as ordinary starting materials for food may be appropriately added, including glycerine, propylene glycol, glycerine fatty acid esters, polyglycerine fatty acid esters, sucrose fatty acid esters, sorbitan fatty acid esters, propylene glycol fatty acid esters, gum arabic, dyestuffs, flavors, preservatives and the like.

The method of preparing the enteral nutrient of the present invention is not specifically limited. Based on the composition illustrated hereinabove, there can be used procedures described in examples appearing hereinafter, or an existing method hitherto known as a procedure of preparing an enteral nutrient, or a method newly provided in the future.

The enteral nutrient of the present invention may be one that is in a state of being filled such as in a container. In this case, there can be adopted a method wherein a preparation is aseptically filled after preliminary thermal sterilization (e.g. a method using in combination a UHT sterilization method and an aseptic filling method), or a method of thermal sterilization along with a container after filling in the container (retort sterilization method, boiling sterilization method). It will be noted that the UHT sterilization method can be carried out according to known procedures including direct heating processes such as a steam injection process wherein steam is directly blown into a drink and a steam infusion process wherein a drink is heated by steam infusion, or indirect heating processes making use of a surface heat exchanger such as a plate, tube or the like. In any of the sterilization processes, heat treatment at 130 to 150°C for about 2 to 120 seconds is preferred. With the case of the retort sterilization, heat treatment at 110 to 125°C for about 4 to 30 minutes is preferred. With the boiling (high temperature-normal pressure) sterilization, the heat treatment is preferably carried out at a pH of liquid enteral nutrient of not higher than 4.6 at 70 to 95°C for about 5 to 20 minutes. Moreover, the thermal sterilization may be carried out in an atmosphere of an inert gas such as nitrogen, if necessary.

Although the container accommodating the enteral nutrient of the present invention is not specifically limited, such a form that allows easy intake on the part of patient is preferred. For example, plastic bottles, PET bottles, cartocans, paper containers such as tetra classic, aluminum pouches, metallic cans or the like may be cited.

The materials for the container used may include soft synthetic resins (e.g. plasticized vinyl chloride resin, polyurethane resins, various types of polyolefin resins including polyethylene (PE) resin, polypropylene (PP) resin, ethylene-vinyl acetate copolymer (EVA), ethylene-alpha-olefin copolymers and the like, polyfluorocarbon, polyimides and the like). These are formed into hermetic containers and soft containers capable of thermal sterilization are favorable. Additionally, containers formed of materials made by laminating an aluminum foil on paper and further a synthetic resin (e.g. polyethylene) on the inner surface side of the container may be used. This laminate container is preferred for the aseptic packing methods.

Besides, resins used such as in medical containers may also be appropriately used. A gas barrier resin layer made of polyethylene terephthalate (PET), polyethylene naphthalate (PEN), ethylene-vinyl alcohol copolymer (EVOH), polyvinylidene chloride (PVDC), polyacrylonitrile, polyvinyl alcohol, polyamide, polyester or the like and a layer having a gas barrier property such as an aluminum foil, an aluminum-deposited film, a silicon oxide film, an aluminum oxide film or the like, provided that when container transparency is required, transparent ones are selected among them, are used in appropriate combination with such a soft synthetic resin as mentioned above, if necessary, for preferred use as a film layer component or components.

### Examples

Next, examples are described to illustrate the invention in more detail, and the invention should not be construed as limited thereto.

### <Examples 1 to 4 and Comparative Examples 1 and 2>

Enteral nutrients of Examples 1 to 4 and Comparative Examples 1 and 2 having amounts of protein indicated in Table 1 and amounts of all components other than protein indicated in Table 4 were prepared in the following way. Initially, agar and a stabilizing agent were added to hot water of 85°C and dissolved well, after which milk serum protein (Alacen 392 (Fonterra Japan Limited)), a soy protein containing about 29% of a peptide fraction having a molecular weight of less than 6000 (Proleena (registered trade name) 900 (Fuji Oil Co., Ltd.)), dextrin (TK-16 (Matsutani Chemical Industry Co., Ltd.)) and superfine sugar were added, followed by further addition of malic acid so as to give a pH of 3.4 to 3.8 and sufficient agitation to provide an aqueous phase. A fat soluble vitamin mix and a glycerine fatty acid ester serving as an emulsifier (Poem W-60, Riken Vitamin Co., Ltd.) were charged into warmed plant oil and dissolved therein to provide an oil phase.

The oil phase was mixed with the aqueous phase and preliminarily emulsified for five minutes by means of a propeller agitator, to which zinc gluconate, copper gluconate, an yeast mix, a seaweed extract, sodium chloride, potassium chloride, sodium dihydrogen phosphate, magnesium chloride, iron citrate, calcium gluconate, a flavor, ascorbic acid, and a water-soluble vitamin mix were added and well agitated. Thereafter, water was added so as to make a total amount of 10 kg thereby obtaining a starting solution. This starting solution was homogenized by means of a high pressure homogenizer at a pressure of 150 MPa and 200 ml thereof was filled in 200 ml cheer pack (registered trade name) ST (Hosokawa Yoko Co., Ltd.) and hermetically closed, followed by boiling sterilization at 90°C for 10 minutes to obtain an enteral nutrient.

### <Comparative Examples 3 to 8>

Enteral nutrients of Comparative Examples 3 to 8 having amounts of protein indicated in Table 2 and amounts of all components other than protein indicated in Table 4 were obtained by repeating the same preparation procedure as in Example 1 except that the soy protein used in Example 1 was changed to a soy protein (Proleena (registered trade name) RD-1, (Fuji Oil Co., Ltd.)) having no peptide fraction having a molecular weight of less than 6000.

### <Example 5>

An enteral nutrient of Example 5 having an amount of protein indicated in Table 3 and amounts of all components other than protein indicated in Table 4 was obtained by repeating the same preparation procedure as in Example 1 except that the soy protein used in Example 1 was changed to a soy protein (Fujipro (registered trade name) CLE, (Fuji Oil Co., Ltd.)) having 31 to 40% of a peptide fraction having a molecular weight of less than 6000.

### <Comparative Example 9>

An enteral nutrient of Comparative Example 10 having an amount of protein indicated in Table 3 and amounts of all components other than protein indicated in Table 4 was obtained by repeating the same preparation procedure as in Example 1 except that the soy protein used in Example 1 was changed to a soy protein (Proleena (registered trade name) 700, (Fuji Oil Co., Ltd.)) having 10 to 19% of a peptide fraction having a molecular weight of less than 6000.

### <Comparative Example 10>

An enteral nutrient of Comparative Example 11 having an amount of protein indicated in Table 3 and amounts of all components other than protein indicated in Table 4 was obtained by repeating the same preparation procedure as in Example 1 except that the soy protein used in Example 1 was changed to a soy protein (Sunlover (registered trade name) 50, (Fuji Oil Co., Ltd.)) having 5 to 9% of a peptide fraction having a molecular weight of less than 6000.

### <Comparative Example 11>

An enteral nutrient of Comparative Example 12 having an amount of protein indicated in Table 3 and amounts of all components other than protein indicated in Table 4 was obtained by repeating the same preparation procedure as in Example 1 except that the soy protein used in Example 1 was changed to a soy protein (Solpea (registered trade name) 400, The Nisshin OilliO Group, Ltd.) having no peptide fraction having a molecular weight of less than 6000.

**[Table 1]**

| | | Example 1 | Example 2 | Example 3 | Example 4 | Comp. Ex. 1 | Comp. Ex. 2 |
|---|---|---|---|---|---|---|---|
| Protein | Soy protein containing a peptide fraction of less than 6000 (g/10 Kg) | 75 | 150 | 300 | 450 | 600 | 750 |
| | Ratio of a peptide fraction of less than 6000 in soy protein | 20 to 30% | | | | | |
| | Soy protein free of a peptide fraction of less than 6000 (g/10 Kg) | - | | - | - | - | - |
| | Soy protein commercial name | Proleena 900 | | | | | |
| | Milk serum protein (g/10 Kg) | 675 | 600 | 450 | 300 | 150 | 0 |
| | Soy protein in total proteins | 10% | 20% | 40% | 60% | 80% | 100% |

**[Table 2]**

| | | Comp. Ex. 3 | Comp. Ex. 4 | Comp. Ex. 5 | Comp. Ex. 6 | Comp. Ex. 7 | Comp. Ex. 8 |
|---|---|---|---|---|---|---|---|
| Protein | Soy protein containing a peptide fraction of less than 6000 (g/10 Kg) | - | - | - | - | - | - |
| | Ratio of a peptide fraction of less than 6000 in soy protein | - | | | | | |
| | Soy protein free of a peptide fraction of less than 6000 (g/10 Kg) | 75 | 150 | 300 | 450 | 600 | 750 |
| | Soy protein commercial name | Proleena RD-1 | | | | | |
| | Milk serum protein (g/10 Kg) | 675 | 600 | 450 | 300 | 150 | 0 |
| | Soy protein in total proteins | 10% | 20% | 40% | 60% | 80% | 100% |

**[Table 3]**

| | | Example 5 | Comp. Ex. 9 | Comp. Ex. 10 | Comp. Ex. 11 |
|---|---|---|---|---|---|
| Protein | Soy protein containing a peptide fraction of less than 6000 (g/10 Kg) | 150 | 150 | 150 | - |
| | Ratio of a peptide fraction of less than 6000 in soy protein | 31 to 40% | 10 to 19% | 5 to 9% | - |
| | Soy protein free of a peptide fraction of less than 6000 (g/10 Kg) | - | - | - | 150 |
| | Soy protein commercial name | Fujipro CLE | Proleena 700 | Sunlover 50 | Solpea 4000 |
| | Milk serum protein (g/10 Kg) | 600 | 600 | 600 | 600 |
| | Soy protein in total proteins | 20% | 20% | 20% | 20% |

**[Table 4]**

| | | |
|---|---|---|
| Carbohydrate | Dextrin | 955 |
| | Superfine sugar | 955 |
| Mineral | Sodium chloride | 3.0 |
| | Calcium gluconate | 83.4 |
| | Magnesium chloride | 43.9 |
| | Disodium hydrogen phosphate | 37.5 |
| | Tripotassium citrate | 30.5 |
| | Potassium chloride | 7.5 |
| | Zinc gluconate | 1.5 |
| | Copper gluconate | 0.1 |
| | Iron citrate | 0.9 |
| Oil | Mixed plant oil | 330 |
| Vitamin | Fat-soluble vitamin mix | 6.3 |
| | Water-soluble vitamin mix | 1.7 |
| | Ascorbic acid | 5.0 |
| Organic acid | Citric acid | 0.8 |
| | Malic acid | 150.7 |
| Trace element | Yeast mix | 2.6 |
| | Seaweed mix | 1.8 |
| Agar | | 30.0 |
| Stabilizing agent | | 56.0 |
| Emulsifier | | 38.0 |
| Flavor | | 6.0 |
| Water | | Appropriate amount |
| Unit: g/10 Kg | | |
| Remarks: common to all the examples and comparative examples | | |

### <Evaluation test 1: presence or absence of coagulation>

A state of each the enteral nutrient was visually observed after dissolution of the soy protein in the preparation of the enteral nutrients of Examples 1 to 5 and Comparative Examples 1 to 11 (but prior to sterilization) and also after boiling sterilization at 90°C for 10 minutes. The results are shown in Tables 5 to 7. It will be noted that in the tables, A indicates no coagulant, B indicates the presence of coagulant, and C indicates a large amount of coagulation. In the examples, no coagulation was observed after or prior to the sterilization.

### <Evaluation test 2: average particle size>

An average particle size of each the enteral nutrient of Examples 1 to 5 and Comparative Examples 1 to 11 was measured by use of a particle size analyzer (SALD 7000, Shimadzu Corporation). The results are shown in Tables 5 to 7. In the respective examples, the average particle size was found to be at 10 µm or below.

### <Evaluation test 3: 50 µm filter permeability>

57 g of water was added to 3 g of each of the enteral nutrients of Examples 1 to 5 and Comparative Examples 1 to 11 to provide a 20-fold diluent, and 50g thereof was taken with a catheter chip syringe (Terumo Corporation) and passed through a milk sediment disk (pore size: 50 µm, Toyo Roshi Kaisha, Ltd.), followed by calculating a permeability (%) based on a residual amount after the passage. The results are shown in Tables 5 to 7. In the respective examples, the permeability was found to be 100%.

### <Evaluation test 4: viscosity>

A viscosity of each the enteral nutrient of Examples 1 to 5 and Comparative Examples 1 to 11 was measured by use of a viscometer (RB80L viscometer, Toki Sangyo Co., Ltd.). The results are shown in Tables 5 to 7. In all the examples, the viscosity was within a range of 1000 to 30000 mPa·s.

### <Evaluation test 5: comparison with a commercial product>

The enteral nutrients of Examples 1 to 5 and Comparative Examples 1 to 11 were compared, according to a triangle difference test, with a commercial product (PG Soft (registered trade name), Terumo Corporation) which was similar to that of Example 1 except that the protein was made of 100% milk serum protein and the amounts of all components other than the protein were indicated in Table 4. It will be noted that enteral nutrients with which the number of examinees who answered correctly was not larger than ten among twenty examinees were judged not significantly different from the commercial product, and nutrients with which the number of correctly answered examinees was not larger than ten were judged significantly different from the commercial product. The results are shown in Tables 5 to 7. The nutrients of Examples 1 to 3 and 5 were equivalent to the commercial product wherein the protein was made up of milk serum protein alone.

### <Evaluation test 6: feeling on the tongue and taste>

The enteral nutrients of Examples 1 to 5 and Comparative Examples 1 to 11 were evaluated with respect to the feeling on the tongue and taste. More particularly, a coarse feeling in the mouth and bitterness inherent to soybean were evaluated by ten panelists. The results are shown in Tables 5 to 7. It will be noted that A indicates that 8 to 10 panelists felt good with respect to both the feeling on the tongue and taste, B indicates that 5 to 7 panelists felt good about both the feeling on the tongue and taste, and C indicates that 0 to 4 panelists felt good about both the feeling and taste. In all the examples, good results were obtained.

**[Table 5]**

| Evaluation Entries | | Example 1 | Example 2 | Example 3 | Example 4 | Comp. Ex. 1 | Comp. Ex. 2 |
|---|---|---|---|---|---|---|---|
| Presence or absence of coagulation | Prior to sterilization | A | A | A | A | A | A |
| | After sterilization | A | A | A | A | C | C |
| Average particle size (µm) | | 7.5 | 9.9 | 9.8 | 10 | 13.5 | 16.5 |
| 50 µm filter permeability (%) | | 100 | 100 | 100 | 100 | 65 | 54 |
| pH | | 3.8 | 3.8 | 3.8 | 4.0 | 4.2 | 4.4 |
| Viscosity (mPa·s) | | 22000 | 20000 | 21000 | 18000 | 11000 | 3500 |
| Evaluation of taste (compared with commercial product) | | no | no | no | yes | yes | yes |
| Evaluation of taste (feeling on the tongue, taste) | | A | A | A | A | C | C |

**[Table 6]**

| Evaluation items | | Comp. Ex. 3 | Comp. Ex. 4 | Comp. Ex. 5 | Comp. Ex. 6 | Comp. Ex. 7 | Comp. Ex. 8 |
|---|---|---|---|---|---|---|---|
| Presence or absence of coagulation | Prior to sterilization | C | C | C | C | C | C |
| | After sterilization | C | C | C | C | C | C |
| Average particle size (µm) | | 18.8 | 19.6 | 21.8 | Unmeasurable | Unmeasurable | Unmeasurable |
| 50 µm filter permeability (%) | | 63 | 43 | 26 | 0 | 0 | 0 |
| pH | | 3.8 | 3.8 | 3.8 | 4.2 | 4.3 | 4.5 |
| Viscosity (mPa·s) | | 22000 | 23200 | 23400 | 19000 | 7800 | 3000 |
| Evaluation of taste (compared with commercial product) | | yes (coarse texture) | yes | yes | Yes | yes | yes |
| Evaluation of taste (feeling on the tongue, taste) | | C | C | C | C | C | C |

**[Table 7]**

| Evaluation items | | Example 5 | Comp. Ex. 9 | Comp. Ex. 10 | Comp. Ex. 11 |
|---|---|---|---|---|---|
| Presence or absence of coagulation | Prior to sterilization | A | A | B | C |
| | After sterilization | A | A | C | C |
| Average particle size (µm) | | 9.2 | 16.6 | 17.4 | 18.7 |
| 50 µm filter permeability (%) | | 100 | 51 | 49 | 49 |
| pH | | 3.8 | 3.8 | 3.8 | 3.8 |
| Viscosity (mPa·s) | | 20500 | 21000 | 22000 | 22500 |
| Evaluation of taste (compared with commercial product) | | no | yes | yes | yes |
| Evaluation of taste (feeling on the tongue, taste) | | A | C | C | C |

### Industrial Applicability

In an enteral nutrient of an acidic type which comprises a protein, a fat, a carbohydrate, a vitamin, a mineral and a food fiber, and has pH in the range of 3 to 4, a soy protein containing 20 to 40% of a peptide fraction having a molecular weight of less than 6000 is formulated in an amount of 5 to 65%, relative to the total proteins. In doing so, although the soy protein is contained as a protein source, there can be provided an enteral nutrient that does not cause an abrupt increase in viscosity and inhomogeneity ascribed to the aggregation or thermal denaturation occurring by heating for sterilization or formulation step and is thus stable, and shows a good taste without giving the sense of bitterness and coarseness inherent to soybean.

## Claims

1. An enteral nutrient which comprises a protein, a fat, a carbohydrate, a vitamin and a mineral and has a pH of 3.0 to 4.5, **characterized in that** said protein includes 5 to 65%, relative to the total protein, of a soy protein containing 20 to 40% of a peptide fraction having a molecular weight of less than 6000.

2. The enteral nutrient as defined in claim 1, which has an average particle size of not larger than 10 µm and is capable of being permeated through a 50 µm filter.

3. The enteral nutrient as defined in claim 1 or 2, which has a viscosity after thermal sterilization of 1000 to 30000 mPa·s.

4. The enteral nutrient as defined in any one of claims 1 to 3, wherein the amount of the soy protein having 20 to 40% of a peptide fraction having a molecular weight of less than 6000 to be formulated is 10 to 60 wt%, relative to the total protein.

5. The enteral nutrient as defined in any one of claims 1 to 4, which further comprises a protein or a peptide selected from the group consisting of milk proteins, animal proteins and peptides prepared from the animal proteins, and plant proteins and peptides prepared from the plant proteins.

6. The enteral nutrient as defined in any one of claims 1 to 5, which further comprises milk proteins.

7. The enteral nutrient as defined in any one of claims 1 to 6, wherein the carbohydrate is dextrin having a Dextrose Equivalent ranging from 8 to 25.

## Patentansprüche

1. Enteraler Nährstoff, welcher ein Protein, ein Fett, ein Kohlenhydrat, ein Vitamin und ein Mineral umfasst und einen pH von 3,0 bis 4,5 aufweist, **dadurch gekennzeichnet, dass** das Protein, relativ zu dem Gesamtprotein, 5 bis 65% eines Sojaproteins beinhaltet, das 20 bis 40% einer Peptidfraktion enthält, die ein Molekulargewicht von weniger als 6000 aufweist.

2. Enteraler Nährstoff, wie in Anspruch 1 definiert, welcher eine durchschnittliche Teilchengröße von nicht größer als 10 µm aufweist und in der Lage ist, durch einen 50 µm-Filter zu dringen.

3. Enteraler Nährstoff, wie in Anspruch 1 oder 2 definiert, welcher nach thermischer Sterilisation eine Viskosität von 1000 bis 30000 mPa·s aufweist.

4. Enteraler Nährstoff, wie in einem der Ansprüche 1 bis 3 definiert, wobei die zu formulierende Menge des Sojaproteins mit 20 bis 40% einer Peptidfraktion, die ein Molekulargewicht von weniger als 6000 aufweist, relativ zu dem Gesamtprotein 10 bis 60 Gew.-% ist.

5. Enteraler Nährstoff, wie in einem der Ansprüche 1 bis 4 definiert, welcher zusätzlich ein Protein oder ein Peptid ausgewählt aus der Gruppe bestehend aus Milchproteinen, tierischen Proteinen und Peptiden, die aus den tierischen Proteinen zubereitet sind, und Pflanzenproteinen und Peptiden, die aus den Pflanzenproteinen zubereitet sind, umfasst.

6. Enteraler Nährstoff, wie in einem der Ansprüche 1 bis 5 definiert, welcher außerdem Milchproteine umfasst.

7. Enteraler Nährstoff, wie in einem der Ansprüche 1 bis 6 definiert, wobei das Kohlenhydrat Dextrin mit einem Dextrose-Äquivalent im Bereich von 8 bis 25 ist.

## Revendications

1. Nutriment à usage entéral qui comprend une protéine, une graisse, un glucide, une vitamine et un minéral et a un pH de 3,0 a à 4,5, **caractérisé en ce que** ladite protéine contient de 5 à 65 %, par rapport à la protéine totale, d'une protéine de soja contenant de 20 à 40 % d'une fraction peptidique ayant un poids moléculaire inférieur à 6 000.

2. Nutriment à usage entéral selon la revendication 1, qui a une taille de particule moyenne inférieure ou égale à 10 µm et est capable de perméation à travers un filtre de 50 µm.

3. Nutriment à usage entéral selon la revendication 1 ou 2, qui a une viscosité après stérilisation thermique de 1 000 à 30 000 mPa.s.

4. Nutriment à usage entéral selon l'une quelconque des revendications 1 à 3, dans lequel la quantité de protéine de soja contenant de 20 à 40 % d'une fraction peptidique ayant un poids moléculaire inférieur à 6 000 à formuler représente de 10 à 60 % en poids, par rapport à la protéine totale.

5. Nutriment à usage entéral selon l'une quelconque des revendications 1 à 4, qui comprend en outre une protéine ou un peptide choisi dans le groupe constitué par les protéines de lait, les protéines animales et les peptides préparés à partir de protéines animales, et les protéines végétales et les peptides préparés à partir de protéines végétales.

6. Nutriment à usage entéral selon l'une quelconque des revendications 1 à 5, qui comprend en outre des protéines de lait.

7. Nutriment à usage entéral selon l'une quelconque des revendications 1 à 6, dans lequel le glucide est une dextrine ayant un équivalent dextrose dans la plage de 8 à 25.
